# EUROPEAN PATENT APPLICATION

(11) **EP 4 609 862 A1**
(43) Date of publication of application: **03.09.2025**
(21) Application number: 23886193.4
(22) Date of filing: 31.10.2023
(51) Int. Cl.: A61K 31/445, A61P 17/00, A61P 17/04, A61K 8/49, A61Q 19/00

(54) **COMPOSITION CONTAINING N-NONYLDEOXYNOJIRIMYCIN FOR SUPPRESSION, ALLEVIATION, PALLIATION, OR TREATMENT OF SKIN ITCHING**

(30) Priority: 01.11.2022 KR 20220143391
(71) Applicant: Cha University Industry-Academic Cooperation Foundation, Pocheon-si, Gyeonggi-do 11160 (KR)
(72) Inventor: CHUNG, Ji-Hyung, Seoul 06285 (KR); LEE, Se-Hee, Incheon 22683 (KR)
(74) Representative: Turner, Craig Robert
(86) International application number: PCT/KR2023/017056
(87) International publication number: WO 2024/096497

(57) **Abstract**

The present invention provides a pharmaceutical composition for the inhibition, alleviation, or treatment of skin itching; and a cosmetic composition for the inhibition, alleviation, or improvement of skin itching, comprising N-nonyldeoxynojirimycin.

## Description

### TECHNICAL FIELD

The present invention relates to a pharmaceutical composition for the inhibition, alleviation, or treatment of skin itching; and a cosmetic composition for the inhibition, alleviation, or improvement of skin itching, comprising N-nonyldeoxynojirimycin.

### BACKGROUND ART

Chronic itching of the skin may be largely divided into a lesion associated with the skin barrier and a lesion associated with abnormal immune responses. The itching associated with the skin barrier, which is a symptom that appears widely from infants to adults, is known that external substances (allergens) easily pass through the stratum corneum to cause sensitization in people with damaged skin barriers, thereby bring about skin itching including atopic dermatitis (Agrawal R and Woodfolk JA, Curr Allergy Asthma Rep, 14:433, 2014). It is known that the itching associated with the skin barrier mainly originates from the epidermal keratinocytes (Kim BE and Leung D, Allergy Asthma Immunol Res, 4:12, 2012; Pastore S et al., Eur J Dermatol, 16:125, 2006). The itching associated with abnormal immune responses is accompanied by various diseases in addition to atopic dermatitis and is difficult to resolve with topical cosmetic materials.

The present applicant has disclosed that certain peptide fragments may be usefully used in a cosmetic composition for inhibiting skin wrinkle formation by facilitating the binding between epidermal cells to promote the expression of junction proteins (Korean Patent Nos. 10-1817244 and 10-1817250, Korean Patent Application No. 10-2022-0024100, etc.).

### DISCLOSURE

### Technical Problem

The present inventors have carried out various cell-based studies to develop the ingredients capable of inhibiting or improving skin itching, for natural product-derived ingredients, particularly various alkaloid derivatives. As a result, the present inventors have found that N-nonyldeoxynojirimycin (NN-DNJ) inhibits the release of β-hexosaminidase and prostaglandin 2 (PGE2) in mast cells in a concentration-dependent manner and also inhibits the itching associated with the skin barrier, thereby having excellent inhibitory, alleviating, improving, or therapeutic activity against skin itching.

Therefore, it is an object of the present invention to provide a pharmaceutical composition for the inhibition, alleviation, or treatment of skin itching, comprising N-nonyldeoxynojirimycin.

It is another object of the present invention to provide a cosmetic composition for the inhibition, alleviation, or improvement of skin itching, comprising N-nonyldeoxynojirimycin.

### Technical Solution

In accordance with an aspect of the present invention, there is provided a pharmaceutical composition for the inhibition, alleviation, or treatment of skin itching, comprising N-nonyldeoxynojirimycin.

In accordance with another aspect of the present invention, there is provided a cosmetic composition for the inhibition, alleviation, or improvement of skin itching, comprising N-nonyldeoxynojirimycin.

In the pharmaceutical composition or the cosmetic composition of the present invention, the skin itching may be a skin itching derived from at least one disease or disorder selected from the group consisting of atopic dermatitis, dry skin, contact dermatitis, urticaria, prurigo nodularis, bacterial infection, viral infection, skin parasitic infection, lichen simplex chronicus, insect bite, and nummular dermatitis. In an embodiment, the skin itching may be a skin itching derived from atopic dermatitis or dry skin.

### ADVANTAGEOUS EFFECTS

It is found by the present invention that N-nonyldeoxynojirimycin inhibits the release of β-hexosaminidase and prostaglandin 2 (PGE2) in mast cells in a concentration-dependent manner and also inhibits the itching associated with the skin barrier, thereby having excellent inhibitory, alleviating, improving, or therapeutic activity against skin itching. Therefore, N-nonyldeoxynojirimycin effectively inhibits both the skin itching associated with the skin barrier and the skin itching associated with abnormal immune responses, and thus can be usefully applied to a pharmaceutical composition for the inhibition, alleviation, or treatment of skin itching; and a cosmetic composition for the inhibition, alleviation, or improvement of skin itching.

### DESCRIPTION OF DRAWINGS

FIG. 1 shows the results obtained by evaluating the inhibitory effects of various alkaloid derivatives against β-hexosaminidase release induced by PMA/A23187 stimulation in RBL-2H3 cells.
FIG. 2 shows the results obtained by evaluating the inhibitory effects according to the concentration-by-treatment of N-nonyldeoxynojirimycin against β-hexosaminidase release induced by PMA/A23187 stimulation in RBL-2H3 cells.
FIG. 3 shows the results obtained by evaluating the inhibitory effects according to the concentration-by-treatment of N-nonyldeoxynojirimycin against PGE2 release induced by PMA/A23187 stimulation in RBL-2H3 cells.
FIG. 4 shows the results obtained by evaluating the inhibitory effects according to the concentration-by-treatment of N-nonyldeoxynojirimycin against IL-6 release induced by TNF-α/IFN-γ stimulation in HaCaT cells.
FIG. 5 shows the results obtained by evaluating the inhibitory effects according to the concentration-by-treatment of N-nonyldeoxynojirimycin against CCL17 release induced by TNF-α/IFN-γ stimulation in HaCaT cells.
FIG. 6 shows the results obtained by evaluating the inhibitory effects according to the concentration-by-treatment of N-nonyldeoxynojirimycin against CCL22 release induced by TNF-α/IFN-γ stimulation in HaCaT cells.

### BEST MODE FOR CARRYING OUT THE INVENTION

The present invention provides a composition for the inhibition, alleviation, improvement, or treatment of skin itching, comprising N-nonyldeoxynojirimycin.

The present inventors have carried out screening the substances for inhibiting the β-hexosaminidase release induced by PMA/A23187 stimulation, using mast cells (RBL-2H3 cells), for various natural product-derived ingredients, particularly various alkaloid derivatives; and have found that N-nonyldeoxynojirimycin (NN-DNJ) has an inhibitory activity against the release of β-hexosaminidase and prostaglandin 2 (PGE2) in mast cells in a concentration-dependent manner. And the present inventors have also found that it effectively inhibits the itching associated with the skin barrier. Therefore, N-nonyldeoxynojirimycin can be usefully applied to a pharmaceutical composition for the inhibition, alleviation, or treatment of skin itching; and a cosmetic composition for the inhibition, alleviation, or improvement of skin itching.

N-nonyldeoxynojirimycin (NN-DNJ), a compound having a structure represented by the following Chemical Formula 1, has a glucosidase inhibitory activity and is known to inhibit hepatic glycogenolysis *in vivo.*

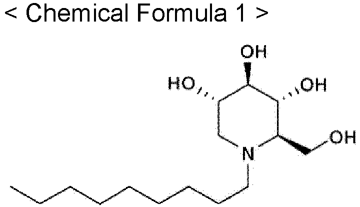

In the composition of the present invention, the skin itching may be a skin itching derived from various chronic skin diseases or disorders. For example, the skin itching may be a skin itching derived from at least one disease or disorder selected from the group consisting of atopic dermatitis, dry skin, contact dermatitis, urticaria, prurigo nodularis, bacterial infection, viral infection, skin parasitic infection, lichen simplex chronicus, insect bite, and nummular dermatitis. In an embodiment, the skin itching may be a skin itching derived from atopic dermatitis or dry skin.

The composition of the present invention may be prepared in the form of a pharmaceutical composition or a cosmetic composition comprising N-nonyldeoxynojirimycin as an active ingredient. For example, the composition of the present invention may be in the form of a pharmaceutical composition for the inhibition, alleviation, or treatment of skin itching, comprising N-nonyldeoxynojirimycin. And, the composition of the present invention may be in the form of a cosmetic composition for the inhibition, alleviation, or improvement of skin itching, comprising N-nonyldeoxynojirimycin.

The pharmaceutical composition may include a pharmaceutically acceptable carrier and may be formulated into a parenteral dosage form, such as a solution, a suspension, an emulsion, a lotion, an ointment, or a lyophilized preparation, according to conventional methods. Preferably, it may be formulated into a formulation for transdermal administration, such as a solution for external use, an emulsion, or an ointment. The pharmaceutically acceptable carrier includes aqueous diluents or solvents such as phosphate buffered saline, purified water, and sterile water and non-aqueous diluents or solvents such as propylene glycol, polyethylene glycol, and olive oil. And, it may include humectants, fragrances, preservatives, etc. as needed. The dose of N-nonyldeoxynojirimycin contained in the pharmaceutical composition may be appropriately selected by a person skilled in the art, depending on the patient's condition, degree of disease, administration route, and administration period. N-nonyldeoxynojirimycin may be administered (e.g., applied) to the skin site with itching e.g., in an amount of 3 µg to 3 mg per day and the administration may be performed once or several times per day.

The cosmetic composition may be in the form of a functional cosmetic composition comprising N-nonyldeoxynojirimycin as an active ingredient. The cosmetic composition may be prepared in various forms according to conventional methods in the field of cosmetic composition. For example, the cosmetic composition may be prepared in forms of cosmetic products, cosmetic solutions, creams, lotions, etc., which may be diluted with a cleansing water, an astringent solution, or a moisture solution, for the use thereof. And, the cosmetic composition may include conventional excipients, such as a stabilizer, a solubilizing agent, vitamins, a pigment, a flavoring agent, which are conventionally used in the field of cosmetic composition. In the cosmetic composition, N-nonyldeoxynojirimycin may be present in an amount effective to achieve the inhibition, alleviation, or improvement of skin itching, for example, in an amount of 0.001 to 10 % by weight, based on the total weight of the composition.

The present invention will be described in further detail with reference to the following examples. These examples are for illustrative purposes only and are not intended to limit the scope of the present invention.

### Example 1: Culture of mast cells (RBL-2H3 cells)

Mast cells (RBL-2H3 cells) were purchased from ATCC (American Type Culture Collection, USA). RBL-2H3 cells were cultured in a DMEM medium (Dulbecco's Modified Eagle's medium (high glucose), Hyclone, USA) containing 10% fetal bovine serum (FBS) and 1% penicillin/streptomycin at 37°C in a 5% CO₂ incubator.

### Example 2: Evaluation of inhibitory effects against β-hexosaminidase release in RBL-2H3 cells

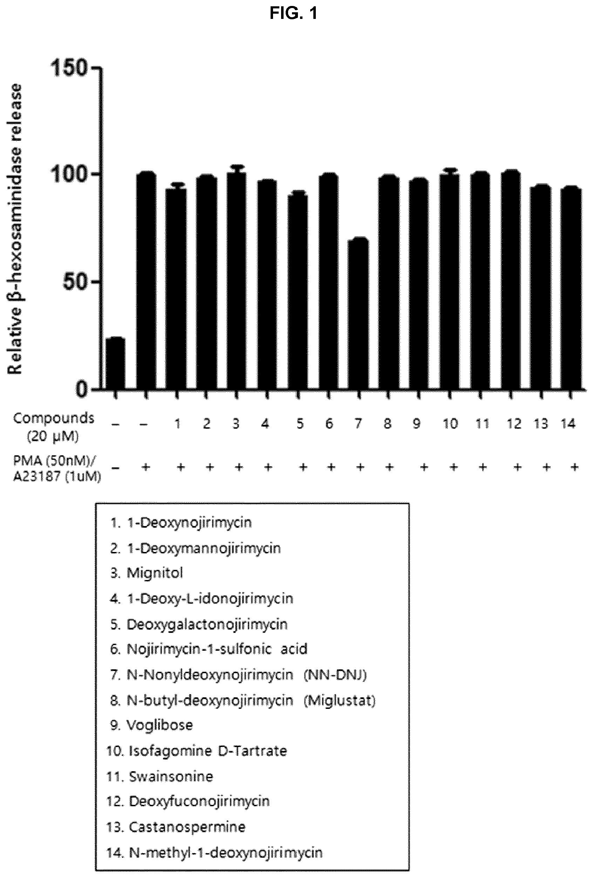

In order to evaluate the inhibitory effects against degranulation of RBL-2H3 cells, β-hexosaminidase release was measured for various compounds. Specifically, the cultured RBL-2H3 cells were seeded at 2x10⁵ cells/well in each well of a 24-well plate for cell culture and cultured in a DMEM medium containing 10% FBS and 1% penicillin/streptomycin. After 24 hours, the existing medium was removed. The cells were washed twice with an extracellular buffer (5 mM KCI, 119 mM NaCl, 25 mM PIPES, 0.4 mM MgCl₂, 1 mM CaCl₂, 40 mM NaOH, 5.6 mM glucose, 0.1% BSA, pH 7.2) and the medium was replaced with the extracellular buffer. Each test material [1-Deoxynojirimycin, 1-Deoxymannojirimycin, Miglitol, 1-Deoxy-L-idonojirimycin, Deoxygalactonojirimycin, nojirimycin-1-sulfonic acid, N-nonyldeoxynojirimycin (NN-DNJ), N-butyl-deoxynojirimycin (Miglustat), Voglibose, Isofagomine D-Tartrate, Swainsonine, Deoxyfuconojirimycin, Castanospermine, and N-methyl-1-deoxynojirimycin] was treated at 20 µM and cultured for 1 hour at 37°C in a 5% CO₂ incubator. The cells were treated with 50 nM of Phorbol 12-myristate 13-acetate (PMA) and 1 µM of A23187 (Merck, Germany) which is a calcium ionophore and incubated for an additional 30 minutes. After the well plate was left in an ice bath at 4°C for 10 minutes to terminate the degranulation reaction, the supernatant was mixed at the same ratio with a substrate buffer (1 mM 4-p-nitrophenyl N-acetyl-beta-D-glucosamine, 0.05 M sodium citrate, pH 4.5) to react for 1 hour at 37°C in a 5% CO₂ incubator. After the reaction was terminated by adding a stop solution (0.1 M carbonate buffer, pH 10), the absorbance was measured at 405 nm using a microplate reader. The results are shown in FIG. 1. From the results of FIG. 1, it can be confirmed that N-nonyldeoxynojirimycin remarkably inhibited the β-hexosaminidase release compared to other alkaloid analogues, thereby exhibiting a remarkably excellent inhibitory effect against degranulation of RBL-2H3 cells.

### Example 3: Evaluation of inhibitory effects against β-hexosaminidase release, according to the treatment concentrations of N-nonyldeoxynojirimycin, in RBL-2H3 cells

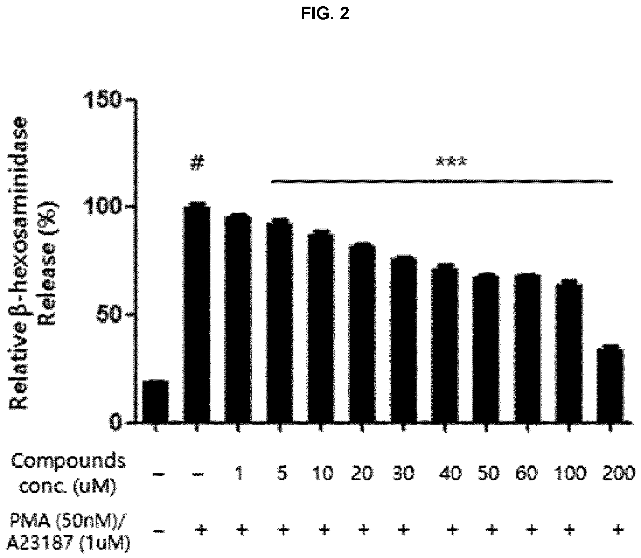

The β-hexosaminidase release was measured in the same manner as in Example 2, except that N-nonyldeoxynojirimycin was treated at various concentrations (1 to 200 µM). The results are shown in FIG. 2. From the results of FIG. 2, it can be confirmed that N-nonyldeoxynojirimycin inhibits the β-hexosaminidase release in a concentration-dependent manner.

### Example 4: Evaluation of inhibitory effects against PGE2 release, by the treatment with N-Nonyldeoxynojirimycin, in RBL-2H3 cells

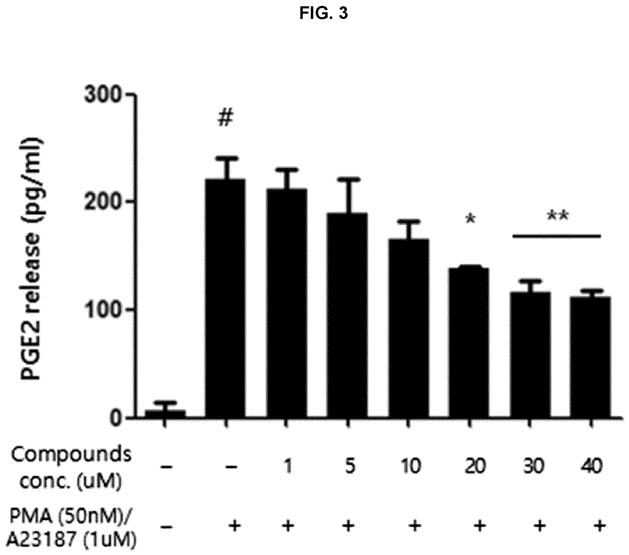

In order to evaluate the inhibitory effects against prostaglandin 2 (PGE2) release of RBL-2H3 cells according to the treatment of N-nonyldeoxynojirimycin, an enzyme-linked immunosorbent assay (ELISA) was performed using a PGE2 ELISA kit (R&D Systems, USA). Specifically, the cultured RBL-2H3 cells were seeded at 1x10⁶ cells/well in each well of a 6-well plate for cell culture and cultured in the same manner as in Example 2. After 24 hours, the existing medium was removed. The cells were washed with PBS and a DMEM medium without FBS was added thereto. After N-nonyldeoxynojirimycin was treated at various concentrations (1 to 40 µM), the cells were cultured for 1 hour at 37°C in a 5% CO₂ incubator. The cells were treated with 50 nM of PMA and 1 µM of A23187 and incubated for an additional 30 minutes. The cell culture mixture was collected in a 1.5 ml tube and then centrifuged at 2,000 rcf. The supernatant thereof was subject to the ELISA analysis using a PGE2 ELISA kit (R&D Systems, USA). After completion of the reaction, the absorbance was measured at 450 nm using a microplate reader. The results are shown in FIG. 3. From the results of FIG. 3, it can be confirmed that N-nonyldeoxynojirimycin inhibited the PGE2 release in a concentration-dependent manner.

### Example 5: Culture of epidermal cells (HaCaT cells)

Epidermal cells (HaCaT cells) were purchased from Thermo Fisher Scientific (USA). HaCaT cells were cultured in a DMEM medium (Dulbecco's Modified Eagle's medium (high glucose), Hyclone, USA) containing 10% fetal bovine serum (FBS) and 1% penicillin/streptomycin at 37°C in a 5% CO₂ incubator.

### Example 6: Evaluation of inhibitory effects against IL-6, CCL17, and CCL22 releases, by the treatment with N-Nonyldeoxynojirimycin, in HaCaT cells

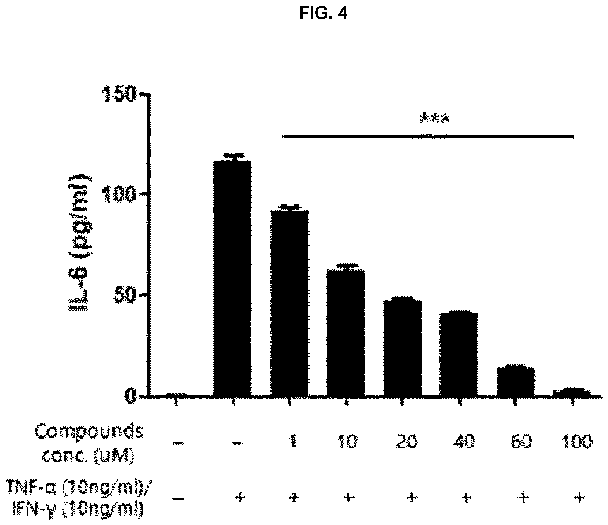

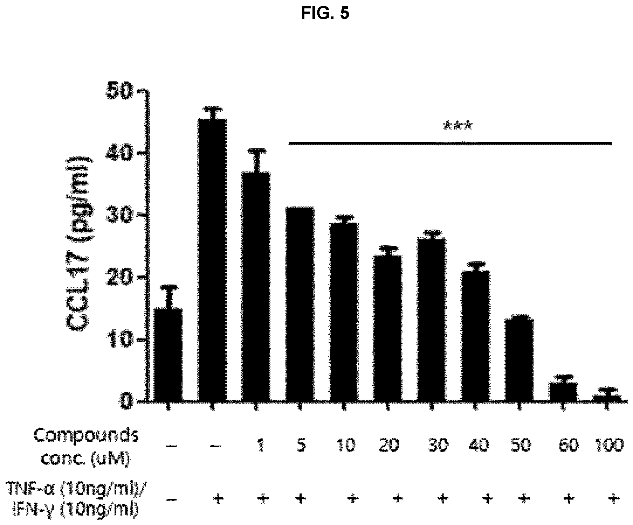

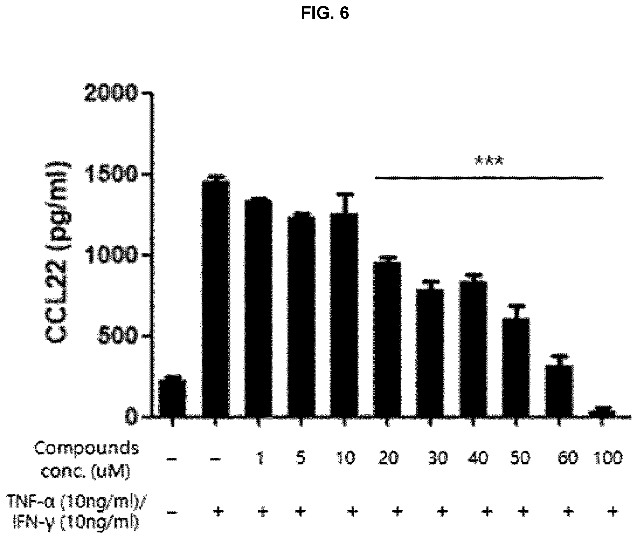

In order to evaluate the inhibitory effects against IL-6, CCL17, and CCL22 release induced by TNF-α and IFN-γ stimulation in HaCaT cells, an epidermal cell model for the itching associated with the skin barrier, we used a IL-6 ELISA kit (Abcam, UK), a CCL17 ELISA kit (R&D Systems, USA), and a CCL22 ELISA kit (R&D Systems, USA). HaCaT cells were seeded at 1x10⁶ cells/well in each well of a coated 6-well plate for cell culture and cultured in a DMEM medium containing 10% FBS and 1% antibiotics (penicillin/streptomycin). After 24 hours, the existing medium was removed. The cells were washed with PBS and a DMEM medium without FBS was added thereto. After N-nonyldeoxynojirimycin was treated at various concentrations, the cells were cultured for 1 hour at 37°C in a 5% CO₂ incubator. The cells were treated with TNF-α and IFN-γ at 10 ng/ml each and then incubated for an additional 24 hours. The cell culture mixture was collected in a 1.5 ml tube and then centrifuged at 2,000 rcf. The supernatant thereof was subject to the ELISA analysis. After completion of the reaction, the absorbance was measured at 450 nm using a microplate reader. The results are shown in FIGs. 4, 5, and 6, respectively. From the results of FIGs. 4, 5, and 6, it can be confirmed that N-nonyldeoxynojirimycin inhibited the IL-6, CCL17, and CCL22 releases induced by itching-inducing stimuli TNF-α/IFN-γ in the epidermal cells, in a concentration-dependent manner.

The present application was carried out under the support of the Gyeonggi-do Regional Research Center (GRRC) (Gyeonggi-do Northeastern Regional Specialized Resources Advanced Center - Health and Functional Food Development of Functional Kimchi and Taemyungcheong beverage).

## Claims

1. A pharmaceutical composition for the inhibition, alleviation, or treatment of skin itching, comprising N-nonyldeoxynojirimycin.

2. The pharmaceutical composition according to claim 1, wherein the skin itching is a skin itching derived from at least one disease or disorder selected from the group consisting of atopic dermatitis, dry skin, contact dermatitis, urticaria, prurigo nodularis, bacterial infection, viral infection, skin parasitic infection, lichen simplex chronicus, insect bite, and nummular dermatitis.

3. The pharmaceutical composition according to claim 1, wherein the skin itching is a skin itching derived from atopic dermatitis or dry skin.

4. A cosmetic composition for the inhibition, alleviation, or improvement of skin itching, comprising N-nonyldeoxynojirimycin.

5. The cosmetic composition according to claim 4, wherein the skin itching is a skin itching derived from at least one disease or disorder selected from the group consisting of atopic dermatitis, dry skin, contact dermatitis, urticaria, prurigo nodularis, bacterial infection, viral infection, skin parasitic infection, lichen simplex chronicus, insect bite, and nummular dermatitis.

6. The cosmetic composition according to claim 4, wherein the skin itching is a skin itching derived from atopic dermatitis or dry skin.
